# EUROPEAN PATENT APPLICATION

(11) **EP 1 696 237 A1**
(43) Date of publication of application: **30.08.2006**
(21) Application number: 05101353.0
(22) Date of filing: 23.02.2005
(51) Int. Cl.: G01N 33/68

(54) **Diagnosis of dry-eye syndrome by SELDI analysis of proteins in tears**

(71) Applicant: Rescom GmbH, 6341 Baar (CH)
(72) Inventor: Grus, Franz, 55218 Mainz (DE); Pfeiffer, Norbert, 55131 Mainz (DE)
(74) Representative: Becker, Konrad

(57) **Abstract**

The invention relates to a method for the diagnosis of dry-eye, related ocular surface diseases and diabetes mellitus based on the protein composition of tears of an individual, using SELDI-TOF-MS (surface-enhanced laser desorption/ionization in time-of-flight mass spectrometry) as a method to profile human tear proteins. Protein or peptide biomarkers for dry-eye disease are selected by comparing the protein profiles of tears from dry-eye patients and healthy controls and analyzing those, using univariate analysis, multivariate statistical techniques and/or artificial neural networks. A multivariate panel of markers is described that classifies the two (or more) groups as accurately as possible.

## Description

### Background of the invention

The number of patients suffering from dry-eye is rapidly increasing. In the United States more than 6% of the population over the age of 40 suffer from dry eye and this prevalence increases up to 15% of people over the age of 60. Dry-eye patients typically suffer from discomfort, burning, irritation, photophobia, and blurred vision and have an elevated risk of ocular infection. In dry-eye patients, some components such as lactoferrin were found to be altered in comparison to healthy subjects.

Previous studies demonstrated that the electrophoretic analysis of tear proteins is able to detect differences in the tear protein profiles between patients suffering from dry-eye syndrome and healthy subjects (Mackie I.A. and Seal D.V., *Br J Ophthalmol.* 1984; 68:321). The stained gel images were digitized, the peak volumes quantified and analyzed by multivariate statistics and artificial neural networks. In these studies, the detection of dry-eye syndrome based on the specific electrophoretic patterns was demonstrated to be superior to detection by clinical standard tests (e.g. Schirmer test (BST) and break-up time (BUT), Grus F.H. *et al., Eur J Ophthalmol.* 1998; 8:90; Grus F.H. and Augustin A.J., *Electrophoresis.* 1999; 20:875; Grus F.H. and Augustin A.J., *Ophthalmologe* 2000; 97:54). Furthermore, altered protein profiles in tears could not only be found in dry-eye, but in some other ocular and systemic diseases (e.g. diabetes mellitus, Herber S. *et al., Adv Exp Med Biol.* 2002; 506:623) and other factors that might interfere with the ocular surface (e.g. contact lenses and smoking (Grus F. H. *et al., Graefes Arch Clin Exp Ophthalmol.* 2002; 240:889). All of these studies provide evidence that specific proteins or peptides in tear film can be used as diagnostic biomarkers for dry-eye, ocular surface diseases, and even systemic disease states such as diabetes mellitus.

Previous investigation of tear film proteins was not limited to one-dimensional gel electrophoretic techniques. In other studies, the tear film proteins were extensively analyzed by high-performance liquid chromatography (Khalil H.A. *et al., Am J Ophthalmol.* 1988; 106:186), two-dimensional gel electrophoresis (Reitz C. *et al., Graefes Arch Clin Exp Ophthalmol.* 1998; 236:894), or other chromatographic approaches (Schmut O. *et al., Graefes Arch Clin Exp Ophthalmol.* 2002; 240:900). 2D electrophoresis is a step forward in proteome research because it offers an improved protein separation compared to 1D electrophoresis. In the first dimension the proteins migrate to their isoelectric point that is specific for each protein. In the second dimension the isoelectrically focused proteins are separated according to their molecular weights. Using this technology, it was possible to analyze and quantify single proteins in tear film and to establish a tear film protein map (Herber S. *et al., loc.cit.;* Reitz C. *et al., loc. cit;* Glasson M.J. *et al., Electrophoresis* 1998; 19:852; Molloy M.P. *et al., Electrophoresis* 1997;18:2811). However, the two-dimensional electrophoretic analysis of proteins is very time-consuming and is limited by problems in reproducibility. This is particularly important when this technique is used for mass-screening of many samples in clinical routine. To eliminate gel-to-gel variation, very complex and tedious procedures to eliminate gel deterioration must be performed to ensure the quality of the spot matching process. Furthermore, the sensitivity of this method limits the analysis to proteins to those larger than approximately 8 to 10 kDa. Considering the increasing demand for mass-screening of protein profiles in tear fluid, a method is needed that overcomes the problems of two-dimensional electrophoretic separations.

SELDI-TOF-MS (Surface Enhanced Laser Desorption/Ionisation Time-of-Flight Mass Spectrometry) ProteinChip Array technology was first described by Hutchens T. and Yip T. *(Rapid Commun Mass Spectrom.* 1993; 7:576) and utilizes chromatographic surfaces to retain proteins based on their physico-chemical characteristics, followed by TOF-MS using a ProteinChip Reader. This technique can be used for a rapid and efficient analysis of body fluids such as sera and cell culture samples (Wellmann A. *et al., Int J Mol Med.* 2002; 9:341; Li J. *et al., Clin Chem.* 2002; 48:1296; Ardekani A.M. *et al., Expert Rev Mol Diagn.* 2002; 2:312; Paweletz C. P. *et al., Dis Markers* 2001; 17:301). Proteins are separated on different array surfaces (e.g. cationic and anionic exchangers, hydrophobic, and metal-ion affinity surfaces). Proteins that are retained on the chromatographic surfaces due to the chosen binding and wash conditions can be easily purified from contaminants such as buffer salts or detergents prior to the analysis by mass spectrometry. Furthermore, the very high sensitivity of ProteinChip Readers is ideal for the analysis of small sample volumes, such as those typically available from tear fluid. An additional advantage of this technique in comparison to 1D and 2D electrophoresis is its ability to screen down to the molecular size of small peptides (approx. 500 Da).

### Summary of the invention

The invention relates to a method for the diagnosis of dry-eye, related ocular surface diseases and diabetes mellitus based on the protein composition of tears of an individual.

The method is characterized in that in a first step, a set of proteins is detected and measured by SELDI-TOF-MS in tears of an individual, and, in a second step, the protein pattern is correlated with corresponding patterns of healthy individuals and dry-eye, related ocular surface diseases and diabetes mellitus patients.

The invention further relates to kits for use in the method of the invention.

### Brief description of the figures

*Figure 1:*
   SELDI-TOF-MS patterns of two healthy subjects (CTRL, A and B) and two dry-eye patients (DRY, A and B) prepared by means of automated sample preparation.
   Top: Grey-scale images of SELDI-TOF-MS data.
   Bottom: Protein mass spectrometry patterns of tears. The intensity of proteins (U) is plotted vs. the molecular weight (Da) in a range up to 35000 Da. The tear samples were prepared on three different array surfaces (H50, CM10, and Q10) and analyzed at the high energy laser setting.
*Figure 2:*
   SELDI-TOF-MS patterns of two healthy subjects (CTRL, A and B) and two dry-eye patients (DRY, A and B) prepared by means of automated sample preparation.
   Top: Grey-scale images of SELDI-TOF-MS data.
   Bottom: Protein mass spectrometry patterns of same tear samples. The intensity of proteins (U) is plotted vs. the molecular weight (Da) in a range up to 40000 Da. The tear samples were prepared on three different array surfaces (H50, CM10, and Q10) and analyzed with the low energy protocol.
*Figure 3:*
   Detection of the two main tear proteins, lysozyme (LYSO) and lipocalin (LIPO), analyzed on three different array surfaces (H50, Q10, and CM10) at high laser energy. Lipocalin is mainly enriched on the anion exchange surface (Q10) at pH 8, whereas it was only slightly bound on the cation exchange surface (CM10). Lysozyme is highly enriched on the CM10 surface.
*Figure 4:*
   The lysozyme peak (LYSO) was detected on a CM10 array with sinapinic acid as the energy absorbing molecule and acquired using low laser intensity. The abundance of this protein was decreased in the dry-eye group (P value < 0.001).
   Top: Box-and-whisker plots of the LYSO concentrations. ("AVG" = mean abundance; "SE" = standard error; "SD" = standard deviation.
   Bottom: averaged group data vectors, representing an "average" SELDI-TOF-MS spectrum, and their corresponding standard errors are plotted against molecular weight.
*Figure 5:*
   Spectra obtained from pooled control tears of healthy volunteers. These samples were processed and analyzed on different arrays and different days on H50 surfaces. The spectra reveal a large similarity in overall profile with average coefficients of variation (CV) in the 28 - 34% range.
*Figure 6:*
   SELDI-TOF-MS patterns obtained from micro-scale analysis of 2 µl tear fluid sample (microcapillary, MIC) and Schirmer strip (SCH) of a dry-eye patient on a reversed-phase surface (H50). The intensity of the peptides and proteins is plotted vs. the molecular weight (Da), which is separated in two graphs for high- and low-molecular weight spectra. Known main protein peaks could be detected, e.g., lysozyme (1), lipocalin (2), and albumin (3). Although the spectra of both tear samples that were obtained by different sampling techniques are quite similar, there are small differences such as an increased number of very small peaks in the lower molecular weight range.
*Figure 7:*
   The intensities of the 4020 Da (NCAPP4) and 4050 Da (PRP4) peaks are decreased in dry-eye patients (P<0.001). Some patients showed only one protein, while others showed both biomarkers. The percentage of patients showing these biomarkers was calculated. The 2 biomarkers are highly present in healthy subjects, whereas the NCAPP4 biomarker and the combination of both biomarkers are less frequently found in dry-eye patients than the PRP4 biomarker alone.
*Figure 8:*
   Top: Statistical distribution for the C-terminal fragment of alpha-1-anti-trypsin as a significant biomarker. For this biomarker, the tear sample was fractionated on 30 kDa cut-off membrane (YM-30). The 4136 Da protein was found in the retentate. The protein was bound to a CM-10 array and identified by the direct sequencing by MS/MS.
   Bottom: ROC (responder operating characteristic) curve showing the diagnostic power of the artificial neural network. The tear protein patterns (comprising the seven protein of Table 1) of dry-eye patients could be differentiated from healthy controls with an area under curve of r = 0.93. x-axis: 1 minus specificity (SPEC), y-axis: sensitivity (SENS).

### Detailed description of the invention

The invention relates to a method for the diagnosis of dry-eye, related ocular surface diseases and diabetes mellitus based on the protein composition of tears of an individual. In essence the invention concerns the use of SELDI-TOF-MS (surface-enhanced laser desorption/ionization in time-of-flight mass spectrometry) as a method to profile human tear proteins. Protein or peptide biomarkers for dry-eye and corresponding diseases are selected by comparing the protein profiles of tears from patients and healthy controls and analyzing those, using univariate analysis, multivariate statistical techniques and/or artificial neural networks.

The procedure is explained in detail for dry-eye disease, but applies equally to other ocular surface diseases, in particular ocular pathologies, such as alterations in tear film composition associated with symptoms similar to dry eye resulting from, e.g., contact lens wear, contact lens cleaning solution or smoking. It likewise applies to diabetes mellitus.

A multivariate panel of markers is described that classifies the dry eye patients and healthy control groups as accurately as possible.

The method for the diagnosis of dry-eye, related ocular surface diseases and diabetes mellitus is characterized in that the marker proteins are measured by SELDI-TOF-MS in tears of an individual, and the marker protein pattern correlated with corresponding patterns of healthy individuals and known dry-eye, related ocular surface diseases and diabetes mellitus patients.

The invention also relates to the method of comparison of complex protein patterns by calculation, e.g. a method of comparison of complex protein patterns by calculation wherein a pattern of marker proteins of an individual is compared with the corresponding marker protein pattern of healthy individuals and of dry-eye patients or patients with related ocular surface diseases and diabetes mellitus.

It may be noted that the invention relates to a comparison of a marker protein pattern, i.e. a complex mixture of a number of proteins both known and unknown. The number of marker proteins may be between 3 and 50 marker proteins, for example in the range of 10 to 30 marker proteins, or, preferably, in the range of 5 to 10 marker proteins, most preferred 7 marker proteins as described in more detail herein below. It is of no relevance for the method of the invention whether the particular marker proteins are properly characterized and what there putative function is, since the procedure relies only on a molecular mass comparison and/or other characteristics of the protein/peptide fragments.

Proteins or peptides are found that are differentially expressed between healthy subjects and those with the dry-eye syndrome, related ocular pathologies and diabetes mellitus. SELDI-TOF-MS ProteinChip Array analysis is very useful for the analysis of tear proteins. This technique allows for straightforward and rapid sample analysis because very small sample volumes can be directly applied to the ProteinChip Array surfaces and the process can easily be automated for high-throughput analysis. The technique has been widely used for the analysis of blood samples and cell culture media. In the present invention this technique is applied to the analysis of tear-fluid proteins.

Proteins and peptides play an important role in preserving the integrity and stability of the ocular surface. Furthermore, the tear film is involved in defense against bacterial and viral infections. It is known that changes in the tear film proteins may be used as biomarkers for dry-eye disease (Grus F.H. *et al., Eur J Ophthalmol.* 1998; 8:90). The electrophoretic techniques used in earlier studies did not allow for the screening of smaller proteins (<9 kDa) and peptides. These peptides, as well as the larger proteins, play an important role in the physiological functions of the tear film. They mediate inflammatory processes, and act as growth factors. The SELDI-TOF-MS technology allows for a sophisticated analysis of peptides, because this technique has its sensitivity maximum in the molecular range between 500 - 25000 Da.

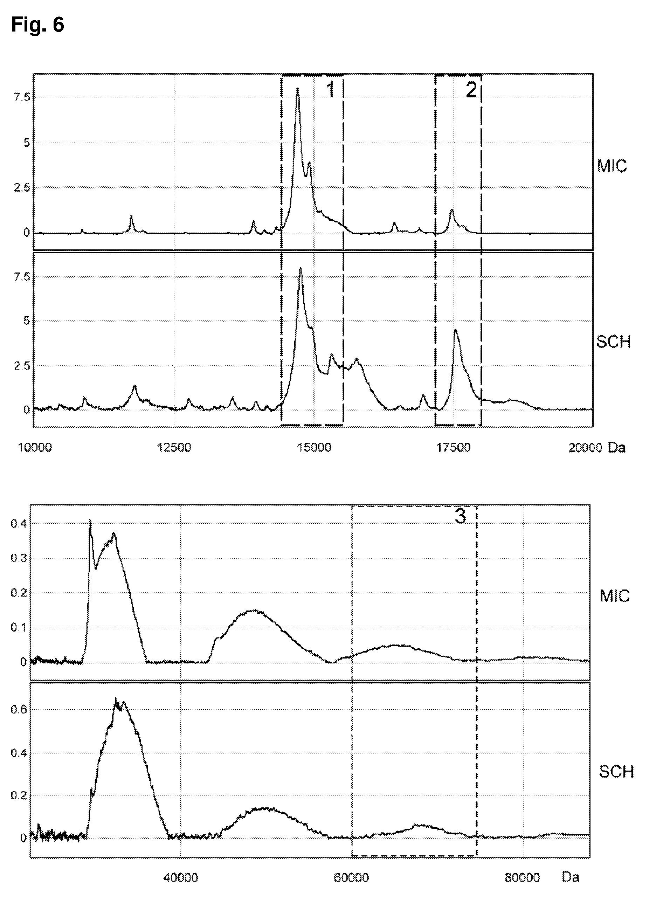

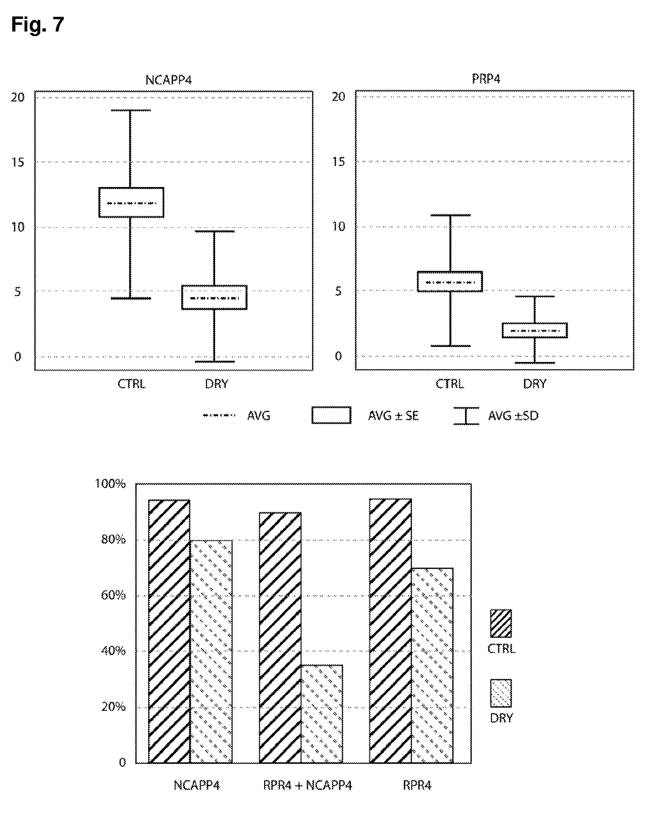

For diagnostic purposes it is desirable to have a platform suitable for screening large numbers of samples. An approach based on an automated robotic sample preparation workstation and a SELDI-TOF-MS ProteinChip Reader equipped with an AutoLoader fulfils this desirable criterion, and is therefore preferred. Both Schirmer strips or microcapillary samples may be used. In order for the volume of the samples to be large enough to be handled by robotic workstations, Schirmer strips are preferred. SELDI-TOF-MS protein profiles of Schirmer strip extracts and those of microcapillary tear samples are essential equivalent, as demonstrated in a direct comparison from a few patients (Figure 6). Similar patterns are found in the tears obtained by both sampling techniques, and there is no indication that proteins could be lost during elution from the strips. Furthermore, an increase of small protein and peptide peaks in the lower molecular weight range are detected from Schirmer strip samples as compared to microcapillary samples. The use of Schirmer strips in the method of the invention is preferred. The inclusion of additional protein and peptide markers from the Schirmer strips will allow better distinction between the different kinds of dry-eye disease, e.g. recognizing Meibomian gland disease, leading to a more differentiated treatment of the disease. The use of Schirmer strips also increases the compliance of physicians. It will not be possible to establish routine clinical use of protein profiling for dry-eye disease and related diseases if the physician has to use a microcapillary that requires up to 15 minutes obtaining a few microliters of tears from severe dry-eye patients. But, if they only have to freeze the Schirmer strips they are already using to determine the BST, they might consider performing this protein profiling.

In proteomics, high-throughput analysis has been hampered by the fact that the most common techniques for protein research, i.e. two-dimensional electrophoresis, are very time-consuming and suffer from poor reproducibility. Furthermore, tedious alignment procedures are necessary to ensure that multiple gels are correctly matched for comparison of protein patterns. In comparison to 2D electrophoresis, the SELDI-TOF-MS technology provides much more precise mass information, allows on-chip protein modifications and protein-protein interaction studies, and enables protein identification. Furthermore, it allows the simple analysis of peptides and is highly sensitive, with just a few µl of sample needed for analysis. Using the preferred automated approach of the invention, it is possible to analyze 96 samples in approximately 4 to 5 hours. In comparison, the two-dimensional electrophoretic analysis will run overnight, the staining procedure will take several hours, and the quantitative analysis is an extremely time-consuming process involving digitization of gels, peak detection, and matching of the 2D gels. Furthermore, these steps include several sources for very common systematic errors. The SELDI-TOF-MS method of the invention allows one to consistently characterize complex spectra of proteins and peptides. The most complex spectra are found in the range <25 kDa, mirroring the sensitivity of the instrument.

Another advantage of the SELDI-TOF-MS technology is the possibility to use different chromatographic surfaces. Depending on what array surface is chosen, protein and peptides can be enriched or selectively washed away from the surface due to their biochemical properties. Lysozyme significantly decreased in dry-eye patients is best detected on the CM10 arrays. In contrast, lipocalin is detected on H50 and Q10 chips.

The primary objective of the invention is the exploitation of differences in the protein and peptide expression in the tears of patients with dry-eye disease, related ocular surface diseases and diabetes mellitus versus healthy control individuals. Many clinical trials in the field of dry-eye rely mainly on the subjective symptoms of patients and do not succeed because of the lack of objective parameters to score the disease and to determine effect of treatment. Thus, the development of tear protein profiling could lead to more clearly defined disease specific protein biomarkers and biomarker patterns.

After detection, the protein pattern is read in a digital image analysis system or other device for digitization, and subsequently analyzed by multivariate statistical techniques, e.g. analysis of discriminance, classification/regression trees, and/or artificial neural networks. Artificial neural networks learn from experience, not from programming. They are fast, tolerant of imperfect data, and do not need formulas or rules. Artificial neural networks are able to generalize and extract consistent features of patterns used to train them. In the present invention, the artificial neural network is "trained" to recognize the protein pattern of dry-eye patients. The preferred artificial neural network used in the invention is the multiple layer feedforward network (MLFN) with the back propagation training algorithm. This kind of network is typically formed by three layers: The input layer receives information from the "external world". The output layer presents the results to the connected devices. A layer of hidden neurons is sandwiched between them. Networks are trained by presenting known samples to them. The network attempts to change the function (weight) of each neuron until all training samples are classified correctly

Other types of artificial neural networks may be used, e.g. self-propagation procedures, probabilistic neural networks, other kind of training algorithms, pruning techniques, and genetic algorithms.

These data analysis techniques differentiate between healthy individuals and dry-eye or related disease patients by considering the whole protein pattern of each patient. By comparing the protein pattern of a patient with suspected dry-eye problem with the protein pattern in samples of other dry-eye patients and healthy individuals, the method can calculate to which clinical group the protein pattern of a patient with unknown pathology reveals the highest similarity.

The method of the invention not only includes the computational techniques as demonstrated herein, but also similar technologies, e.g. the use of other pattern matching techniques, other classifying statistical techniques or other methods to acquire the protein pattern data of individuals.

The method of this invention uses the increase or decrease of proteins in tears of diseased patients, compared to controls, as "biomarkers" for the diagnosis of the disease. This implies that knowledge about the identity of those proteins is not needed for a reliable diagnosis of the disease. No single protein marker reliably distinguishes dry-eye, related ocular surface diseases and diabetes mellitus patients from healthy individuals.

Although the method is described for the diagnosis of dry-eye, related ocular surface disease and diabetes mellitus patients, it can also be used for the assessment of a (healthy) individual's risk for developing dry-eye problems. Furthermore, the described method is useful for assessment of progression and/or severeness of the dry-eye or related ocular surface disease, or for the success of a therapeutic treatment. For that purpose the change in the protein pattern over time and corresponding computational and pattern matching techniques are used. "Method of diagnosis" as described herein therefore not only means the determination that a subject suffers from dry-eye, related ocular surface disease or diabetes mellitus. It likewise means a method for the assessment of the risk for acquiring such a disease, the assessment of progression of the disease, and the assessment of the success of a therapeutic treatment.

In a particular embodiment of the invention, samples were prepared and then the raw spectral data were acquired and processed as described in the Examples. Cluster lists were generated for each condition (low and high laser energy settings) and for each surface (CM10, H50, and Q10) using Ciphergen's "Biomarker Wizard" software. For each of these cluster lists, a multivariate analysis of discriminance was performed which defined a total of approximately 50 peaks with significant differences between the tear protein profiles of CTRL (healthy individuals) and DRY (dry-eye patients) (P<0.01). The objective of protein profiling is to distinguish healthy from disease states based on protein patterns, und use this fact for diagnostic purposes. Since SELDI-TOF-MS is especially sensitive in the low molecular weight range of proteins, it is preferable to differentiate between healthy individuals (CTRL) and dry-eye patients (DRY) based on peptides and small proteins. Thus, the diagnosis is preferably based on biomarkers with a molecular weight lower than lysozyme (approx. 14 kDa). An additional analysis of discriminance with molecular weights smaller 14 kDa reveals a statistically significant difference between CTRL and DRY groups using the biomarker candidates from all conditions and surfaces simultaneously (P<0.0000001). Table 1 lists the first 7 peaks that had statistically the most important effect on the separation between both groups in the analysis of discriminance and were proven not to be MS artifacts such as double charged ions. A set of these 7 proteins is most preferred.

**Table 1**

| | Abundance: | | |
|---|---|---|---|
| Mol. weight | CTRL | DRY | Identification |
| | | | |
| 3700 Da | 3.64 | 1.42 | not identified |
| 3916 Da | 0.18 | 2.5 | not identified |
| 4027 Da | 11.98 | 4.49 | NCAPP4 |
| 4052 Da | 5.62 | 1.78 | p16378 Proline Rich Protein 4 (PRP4) |
| 4136 Da | 0.53 | 0.98 | p01009 alpha-1-Antitrypsin, C-terminal fragment |
| 5792 Da | 0.53 | 0.21 | p02814 Proline Rich Protein 3 (PRP3) |
| 10834 Da | 012 | 0.36 | p05019 Calgranulin A |

Furthermore, some other important biomarkers, which are useful for the diagnosis and follow-up of dry-eye and/or other ocular surface diseases are listed below:
- Several other (smaller) fragments of the members of the Proline-rich protein families
- Fragments of the main tear proteins Lipocalin (LIPO, 17445.50 Da) and Lysozyme C (LYSO, 14700.67 Da)
- Beta-2-microglobulin P61769; 11731.17 Da
- Prolactin induced protein P12273: 16572 und 13522 Da
- Cystatin P01040; 16214 Da and 14188 Da

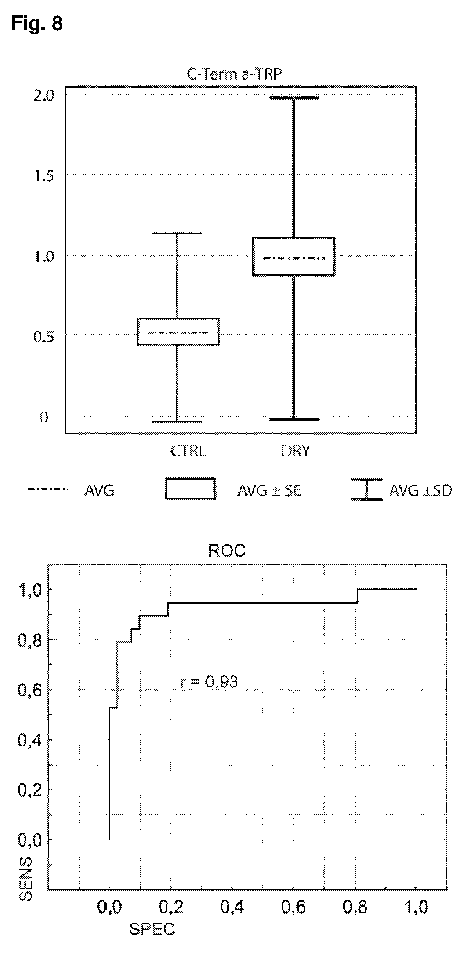

Using this multi-marker panel as input, an artificial neural network was trained using a training data set. The performance of the trained net was assessed using a test data set. Figure 8 shows a ROC curve with an area under the curve of 0.93 and a specificity and sensitivity of approximately 90% for each. Although it is best to use a marker protein pattern comprising all the seven marker proteins of Table 1, the invention may also be performed with a different protein pattern which comprises these 7 proteins and further marker proteins, or which comprises only 4, 5 or 6 of the mentioned seven marker proteins and optionally further marker proteins, for example those listed above.

Some of the biomarkers are down-regulated in dry-eye disease while others are upregulated. Thus, both dry-eye disease and the aqueous deficient dry-eye cannot be described as a simple reduction of the total amount of tear proteins.

Several of the biomarker candidates of the multi-marker panel were enriched and identified using tandem mass spectrometry. The biomarker at 5792 Da was identified as Proline rich protein 3 (PRP3). Biomarkers at 4020 and 4050 Da were detected on the CM10 surface at low laser energy. There was a significant decrease of both peptides in dry-eye patients (P<0.000001). The 4050 Da biomarker was identified as C-terminal fragment of Proline rich protein 4 (PRP4, amino acids 85-118). The 4020 Da biomarker was identified as a variant of the same peptide, the C-terminal fragment of Nasopharyngeal carcinoma-associated Proline-rich protein 4 (NCAPP4, amino acids 85-118). The only difference between these two peptides is a single amino acid substitution from Arg to Gln. Figure 8 shows an elevation of the 4136 Da biomarker in dry-eye patients. This biomarker was identified as the C-terminal fragment of alpha-1-antitrypsin (amino acids 358-394). Finally, the 10834 Da marker was identified as Calgranulin A.

Two of the seven identified biomarkers are members of the family of Proline-rich proteins (PRP3 and PRP4) and are decreased in dry-eye samples. Proline-rich proteins are thought to mediate protective functions in the eye, such as modulation of the microflora. PRP3 is a highly abundant protein in tears. PRP4 is abundantly expressed in lachrymal gland where it is found in the acinar cells, but not in the intralobular ducts. In contrast to other Proline-rich proteins, PRP4 is expressed in the lachrymal acinar cells, and other anterior exocrine glands. A variant of PRP4 (NACPP4) is also decreased in dry-eye patients, and the percentage of patients having both PRP4 and NACPP4 is less than half in dry-eye patients as compared to controls.

The 10.8 kDa biomarker, identified as Calgranulin A, is increased in dry-eye patients. Calgranulin is expressed in neutrophils and is thought to be an inflammatory marker. The 4136 Da biomarker is the C-terminal fragment of alpha-1-antitrypsin. As an inhibitor of proteases, the major physiological function of this molecule is the protection of tissues against proteolytic destruction. An increase of the C-terminal fragment is observed in dry-eye patients, which could indicate an increase in antitrypsin molecules that were inactivated due to cleavage at the reactive bond by enzymes such as metalloproteinases. In the multi-marker panel of the invention, some of the biomarkers that might provide protective functions are downregulated in dry-eye patients while others, including some inflammatory markers, are upregulated.

The invention further relates to kits for diagnosis of dry-eye disease. Such kits may comprise test strips, materials for "lab-on-a-chip" approaches, ELISA techniques, or any kind of mass spectrometry approaches, biochips, and, optionally, a bundled computer software including the specialized algorithm to detect dry-eye, related ocular surface diseases and diabetes mellitus in the protein pattern of patients. Kits according to the invention may be for use by a doctor or even a patient on its own, or for a professional diagnostic service laboratory center.

While this invention has been particularly shown and described with references to preferred embodiments thereof, it will be understood by those skilled in the art that various changes in form and details may be made therein without departing from the spirit and scope of the invention as defined by the appended claims.

### Examples

*Patients:* From 159 patients, 71 patients were categorized as control subjects (CTRL) and 88 patients were suffering from dry-eye syndrome (DRY). The ophthalmic examination included subjective symptoms, visual acuity, Schirmer test with anesthesia (basis secretory test BST), and biomicroscopy with careful examination of the lid margin and Meibomian glands and tear break-up time. In order to assess the subjective symptoms, the patients' history was taken. Each patient was asked for his subjective symptoms such as burning, itching, foreign body sensation, dryness, and photophobia. Patients were classified as dry-eye if they suffered from symptoms of dry-eye as described above and abnormalities of test dynamics determined by Schirmer (< 5 mm / 5 min). Sjoegren patients are excluded. Only tear substitutes without preservatives were accepted as treatment during the month before examination. In order to focus mainly on aqueous deficient dry-eye, patients with clear Meibomian gland dysfunctions were excluded. The normal controls were matched in age and sex to the dry-eye patients. In all patients, the basis secretory test (BST) was performed and the Schirmer strips stored at -80 °C until use.

### SELDI-TOF-MS Analysis

*Samples:* The first 10 mm of the Schirmer strips were eluted overnight in 500 µl phosphate buffered saline (PBS) with 1% Triton X-100. For automatic handling of all binding and washing steps, a robotic laboratory automation station (BIOMEK 2000, Beckman, USA) was used. The station was extended by an integrated microplate mixer (Micromix 5, DPC, USA), which can hold the ProteinChip® Array BioProcessor (Ciphergen, USA). This BioProcessor can be equipped with 12 ProteinChip Arrays, each having eight spots. Using 2 BioProcessors, up to 192 wells with approximately 250-300 µl volumes are handled by the BIOMEK robotic station per well.

The tear fluid was tested on three different chromatographic surfaces: a weak cation exchange surface (CM10), a strong anion exchange surface (Q10), and a reverse phase surface (H50). All ProteinChip Arrays were pre-treated according to the standard protocols of the manufacturer. Binding buffers were 5% acetonitrile/0.1% trifluoroacetic acid (H50), 20 mM sodium acetate buffer, pH 5 (CM10), and 50 mM Tris, pH 8 (Q10). 20 µl of the Schirmer strip elution and 20 µl of binding buffer were applied to each spot using the BIOMEK. The arrays were incubated on the DPC shaking platform for 2 h and the solution in each well was removed by the BIOMEK. All wells were washed three times with 150 µl binding buffer followed by a wash step with 20 µl distilled water for 5 min to remove buffer salts. After drying, the BIOMEK was used to apply 1 µl of a saturated sinapinic acid solution (energy absorbing molecule) in 50% acetonitrile/0.5% trifluoroacetic acid to each spot. After air-drying, each spot was analyzed in a ProteinChip Reader. Each sample was bound to each array surface in duplicate on separate arrays and BioProcessors.

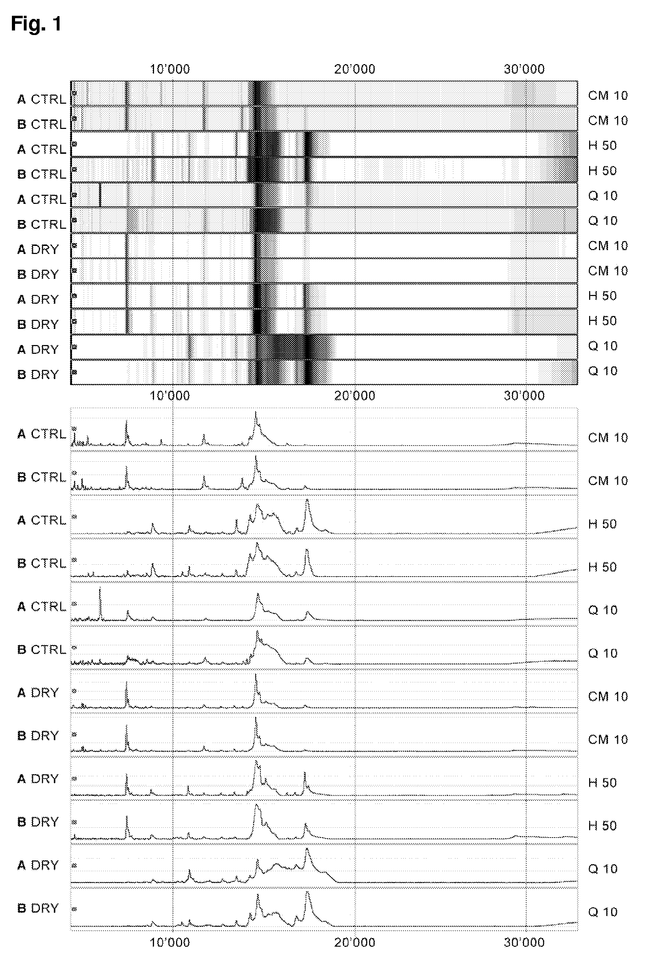

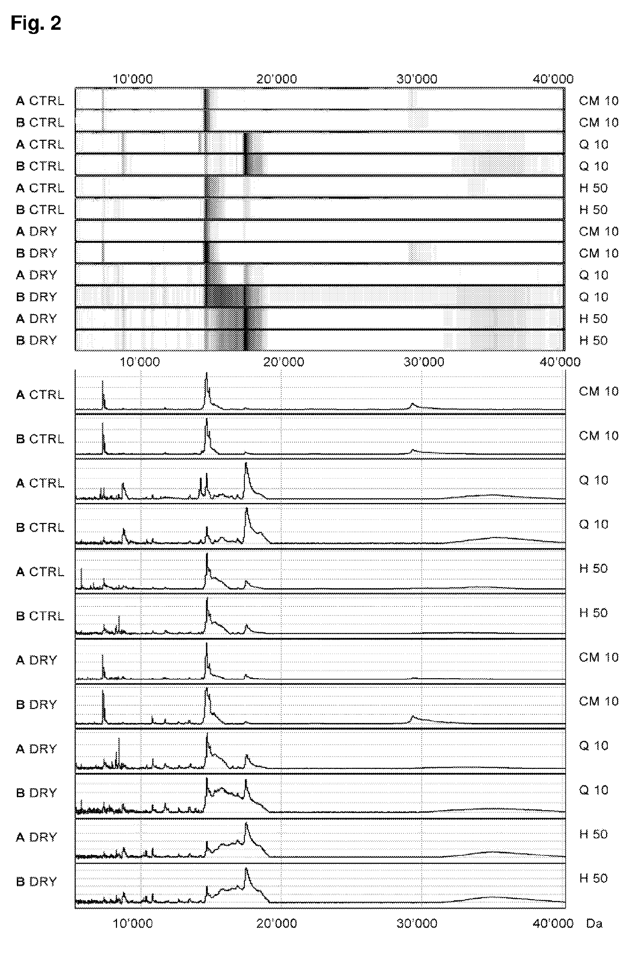

Figure 1 and 2 demonstrate several SELDI-TOF mass spectra of tear protein profiles that illustrate differences in profiles corresponding to different array surfaces and laser intensity conditions. Both figures show mass spectra that were obtained from arrays prepared by the automated robotic station. Figure 1 shows mass spectra of healthy controls (CTRL) and dry-eye patients (DRY) using high laser energy on H50, CM10, and Q10 surfaces. In the gel view, the same protein patterns are shown in a grey-scale presentation, which simulates an electrophoretic separation. In Figure 2, representative spectra of both groups are shown on the three different surfaces for data collected at low laser energy. Both figures provide an insight into the complexity of tear protein profiles in the lower molecular weight range and into the variability from patient to patient.

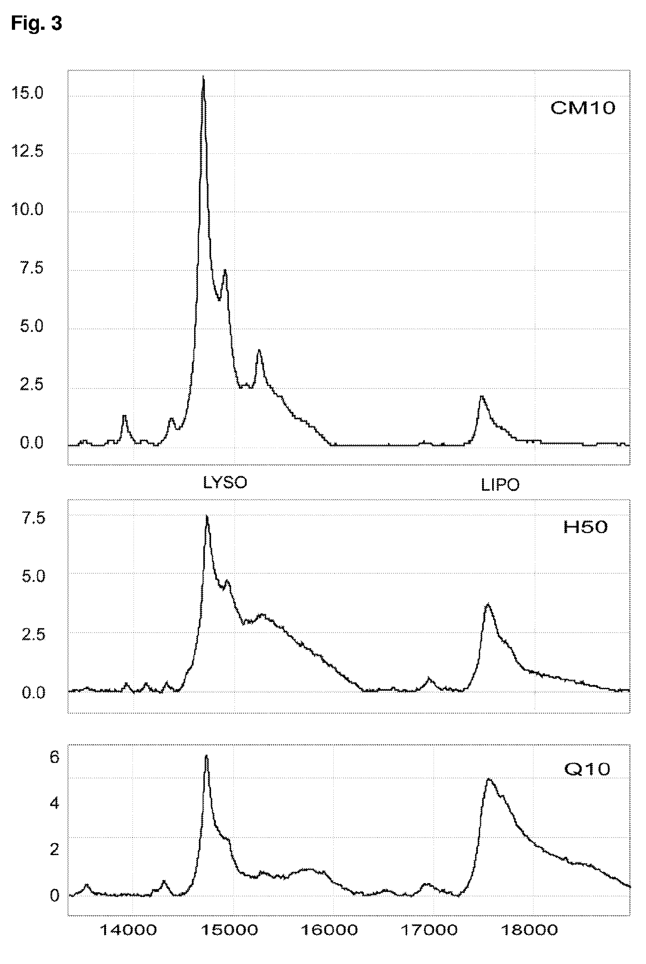

In Figure 3, the elution profiles of two of the main tear proteins, lysozyme and lipocalin, are demonstrated for the different chip surfaces (H50, Q10, and CM10) in the tears of a healthy volunteer. As expected, the protein profiles are different due to the on-chip chromatographic enrichment. The peaks that correspond to lysozyme and lipocalin are marked in Figure 3. While lipocalin is present on the reverse phase (H50) and anion exchange surface (Q10), it was very weakly bound to the weak cation exchanger surface (CM10). Lysozyme, the other most prominent protein peak in tears, shows the strongest peak on the cationic exchange surface (CM10).

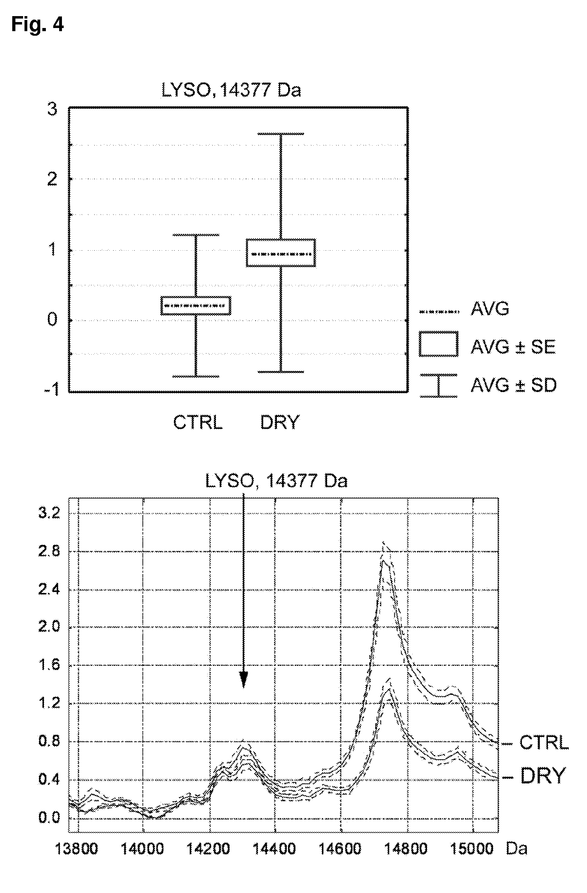

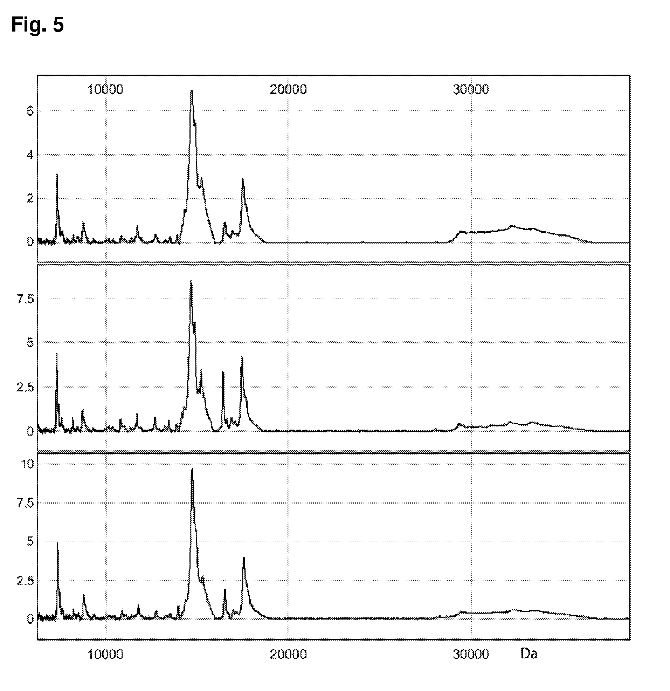

The analysis of the SELDI-TOF-MS spectra confirmed that lysozyme amounts are decreased in tears of dry-eye patients (Figure 4). To evaluate day-to-day variations between the ProteinChip Array profiles, a control (pooled tears from healthy subjects) was processed in every BioProcessor. Figure 5 shows a representative measurement using the same sample in different BioProcessors on different days. Coefficients of variation (CVs) were calculated for this pooled tear sample for all peak clusters and for all surfaces. The average CVs were calculated to be between 28 and 34%. According to the manufacturer, these values are in the same range as those obtained in serum profiling.

*Data Acquisition and Pre-processing:* ProteinChip Arrays were analyzed on a PBS-Ilc ProteinChip Reader equipped with a ProteinChip Array AutoLoader using the ProteinChip Software version 3.2. The AutoLoader is able to analyze up to 24 ProteinChip Arrays (=192 spots) at one time. Each array was read at two laser intensities: Low intensity optimized for lower molecular weight proteins and high intensity for higher molecular weight proteins. The high intensity protocol averaged 195 laser shots from each spot with a laser intensity of 190, a deflector setting of 3000 Da, a detector sensitivity of nine, and a molecular weight detection range of 2000 to 200000 Da. For low intensity measurements, the laser intensity was set at 180 and the deflector set at 1500 Da. The raw data was transferred to the CiphergenExpress™ Data Manager Software version 2.1 (CE) for analysis. The baseline was subtracted using a setting of 12 times the expected peak width. The spectra were calibrated using external calibrants (bovine insulin 5733.6 Da, ubiquitin 8654.8 Da, cytochrome C 12360.2 Da, beta-lactoglobulin 18363.3 Da, and horseradish peroxidase 43240 Da). The low and high laser intensity settings were calibrated separately. Spectral intensities were normalized by total ion current (TIC) to an external normalization coefficient of 0.2. The starting mass for the normalization procedure differed according to the laser energy and the settings in the spot protocol for data acquisition. In this study, the low mass cutoffs were 3000 m/z for arrays at low laser intensity and 8000 m/z for arrays at high laser intensity. Spectra with normalization factors that were more than 100% above the mean were evaluated for possible deletion. They were deleted if their normalization factors were more than double the value calculated for the replicate spectrum. In the case where both spectra were above the cutoff, then both spectra were kept. At least one replicate was retained for every sample.

Automatic peak detection was performed using the settings of 5 times the signal-to-noise ratio for the first pass of peak detection and 2 times the signal-to-noise ratio for the second pass. From these detected peaks, a list of peak clusters was created. The Biomarker Wizard feature in CE generates consistent peak sets across multiple spectra. When comparing a given protein peak across various sample conditions, it is important to obtain an intensity value for every spectrum, even though they may not have been found with a given set of automatic peak detection settings. The Biomarker Wizard operates in two passes. The first pass uses low sensitivity to detect obvious and well-defined peaks. The second pass uses higher sensitivity settings to search for smaller peaks, with the mass values found in the first pass. A peak cluster was created if the given peak was found in 10% of all spectra for an individual condition above the first pass cutoff. The mass window for peak clustering was defined as 0.3% of the peak mass for spectra read at low laser intensity and 2% of the peak mass for spectra read at high laser intensity. A larger mass window is used for the higher MW proteins to account for broadening of peaks due to an increase in protein isoforms.

*Data Analysis:* CiphergenExpress Data Manager Software version 2.1 was used to normalize the spectra, to automatically detect peaks, and to create the peak cluster lists. Because of the inherent variability in the total signal for raw laser desorption/ionization mass spectrometric data, normalized peak intensities were used exclusively for doing statistical analyses. The cluster lists were exported as ASCII files to Statistica (Version 6.2, StatSoft, Tulsa, OK, USA). The cluster lists contained normalized peak intensity values for each sample within a group. Based on these normalized peak intensities, P values based on t-tests and multivariate analysis of discriminance were calculated. Using multi-marker panels of protein biomarkers rather than single biomarkers can generally enhance the separation between clinical groups for diagnostic purposes.

Statistica was used to perform a multivariate analysis of discriminance based on combinations of multiple biomarker peaks. This analysis of discriminance tests the zero hypothesis (namely, that mean biomarker peaks of the different clinical groups derive from a multivariate normally distributed population), and also shows which of the various groups are statistically different. Based on this, discriminant function analysis can be used to determine which variables (peaks at particular molecular weights) generated a significant mean value comparison, or which variables can discriminate between groups. The analysis of discriminance can calculate which variables are the most important to discriminate between the groups and ranks the variables according to their significance. For each condition (surface type and laser energy), an analysis of discriminance was calculated. Each analysis creates a list of peaks that was found to be significantly different between the groups. All of these lists were combined and a final analysis of discriminance was calculated based on the most significant protein biomarkers from all conditions. This final analysis of discriminance selected the seven most important protein biomarkers for best discrimination between the groups.

In order to assess the diagnostic power of this seven biomarker panel, a neural network was trained using these markers as input neurons. The traditional ANN model is the most widely in use today and is the multiple-layer feedforward network (MLFN) with the backpropagation training algorithm (Rumelhart D. *et al., Nature.* 1986; 323:533). The neural network module provided by the Statistica Software (V6.2) was used. The network was trained by the seven biomarker peaks and the output neurons defined with patient was classified as "dry-eye protein pattern" or not. To assess the performance of the network, the dataset was randomly divided in two parts: the first half of the patients and controls was used as training set, the second as a test set. After completion of training, the success of the algorithm was tested using the test data set, which is comprised of patients and controls to which the neural network has not previously been exposed.

To demonstrate the results, the software generates an ROC curve by plotting sensitivity against 1 - specificity (see e.g. Figure 8). A test that perfectly discriminates between two groups would yield a "curve" that coincided with the left and top sides of the plot. A global assessment of the performance of the test (sometimes called diagnostic accuracy) is given by the area under the ROC curve (ROC AUC). A perfect marker with complete group separation would have an ROC AUC value of 1.

*Alternative sampling of tears:* SELDI-TOF-MS protein profiles from tears eluted from Schirmer strips were compared with tears collected by microcapillaries. A tear volume of approximately 5 □ was sampled from one eye of 3 selected patients with a 5 □ glass capillary without touching the lid margins and eye lashes of the patients and stored at - 80°C until use.

H50 ProteinChip Arrays were used for detection of protein profiles from tear fluid samples. For micro-scale analysis, a sample volume of 2 µl tears in 2 µl binding buffer (5% acetonitrile / 0.1 % trifluoroacetic acid) was used. After incubating in a humidity chamber at room temperature for two hours, all spots were washed three times with 5 µl binding buffer followed by a wash step with 3 µl distilled water for 5 min. After air drying, 1 µl of saturated sinapinic acid (energy absorbing molecule) in 50% acetonitrile / 0.5 % trifluoroacetic acid was applied. After air drying, each spot was analyzed by SELDI-TOF-MS as described above (Figure 6).

The x-axis in this figure shows the molecular weight of detected proteins and peptides for two different high and low molecular weight ranges. Several of the main protein peaks predicted from electrophoretic analysis were detected (Grus F.H. and Augustin A.J., *Curr Eye Res.* 1998; 17:636). According to their molecular weights, these proteins are lactoferrin, albumin, lipocalin, lysozyme, and several chains of IgA. In addition to these high molecular weight peaks, a very complex spectrum of peptides was detected in the molecular weight region below 20 kDa in both analyses of tear samples obtained from Schirmer strips and microcapillaries. In general, both collection methods yielded similar results. However, in the protein spectra of the Schirmer strips, an increased number of proteins in the lower molecular weight range could be observed.

*Protein Purification and Identification:* Eluted samples from Schirmer strips were first enriched by combination of cut-off membrane fractionation (YM30 and YM3, Millipore, USA), anion exchange chromatography (Q HyperD® F, Ciphergen, USA), and/or reversed phase chromatography (RPC PolyBio, Ciphergen).

Polypeptides smaller than 4.2 kDa were identified by direct sequencing using a Q-TOF2 tandem mass spectrometer (Micromass, UK) equipped with a Ciphergen PCl-1000 ProteinChip Interface. Proteins were bound from enriched fractions to CM10 or IMAC-Cu arrays. Spectra were collected from 2 to 4.5 kDa in single MS mode. After reviewing the spectra, specific ions were selected and introduced into the collision cell for CID fragmentation. The CID spectral data was submitted to the database mining tools Mascot (Matrix Sciences) or Protein Prospector MS-Tag (UCSF) for identification.

Proteins larger than 4.2 kDa were further purified by SDS-PAGE (16% Tricine gel, Invitrogen, USA). Polyacrylamide gels were stained with Colloidal Blue Staining Kit (Invitrogen). Selected protein bands were excised from the polyacrylamide gel. The gel pieces were washed with 200 □| of 50% methanol/10% acetic acid for 30 min, dehydrated with 100 □| of acetonitrile for 15 min, and extracted with 70 □| of 50% formic acid/25% acetonitrile/15% isopropanol/10% water for 2 h at room temperature with vigorous shaking. 2 □| of extracts were applied to NP20 ProteinChip arrays and re-analyzed using the ProteinChip Reader to confirm m/z values of the excised/extracted proteins. The gel extracts were dried in a Speed-Vac. 20 □| of ammonia solution (concentrated stock diluted 25-fold with water) was added to the tubes, and the solution dried again in a SpeedVac. 20 □| of 2 ng/□| modified Trypsin (Roche Applied Science, USA) in 50 mM ammonium bicarbonate (pH 8) were added to each tube and the samples incubated for 2 - 4 h at 37°C. Peptide identification was performed using the tandem mass spectrometer equipped with a Ciphergen PCI-1000 ProteinChip Interface. MS/MS spectra were submitted to the database mining tool Mascot (Matrix Sciences) for identification.

*Identification of the 5792 Da peak as Proline-rich protein* 3. The 5792 Da protein was detected on CM10 arrays. Approx. 400 µl of Schirmer strip elution was passed through a 30 kDa cut-off membrane (YM-30) and the 5792 Da protein was found in the filtrate. The filtrate fraction was then passed through a 3 kDa cut-off membrane (YM-3) and the 5792 Da protein was found in the retentate. The retentate fraction was dried in a SpeedVac, redissolved in SDS-PAGE buffer and loaded onto a 16% Tricine gel. Coomassie stained bands were extracted and re-profiled on NP20 arrays. In-solution digestion of the 5792 Da protein yielded two unique ions. MS/MS analysis identified both ions as fragments of Proline-rich protein 3. The theoretical MW of the polypeptide is 5809.76 Da. However, the N-terminal Gln of Proline-rich Protein 3 is known to be modified to pyrrolidone carboxylic acid (pyroGlu, -17Da). Therefore, the predicted MW of the protein is 5792.76 Da, which is very close to the observed experimental MW.

*Identification of the 4020 and 4050 Da peaks* as *C-terminal fragment of Nasopharyngeal carcinoma-associated Proline-rich protein 4 (NCAPP4) and Proline-rich protein 4 (PRP4), respectively.* The 4020 and 4050 Da peaks were detected on CM10 arrays using low laser intensity. The 4050 Da biomarker was identified as the C-terminal fragment of Proline-rich protein 4 (PRP4) and the 4020 Da biomarker was identified as the C-terminal fragment of Nasopharyngeal carcinoma-associated Proline-rich protein 4 (NCAPP4). Amino acid alignment of PRP4 and NCAPP4 sequences shows that the two proteins are almost identical with two amino acid differences in position 78 and 102.

## Claims

1. A method for the diagnosis of dry-eye, related ocular surface diseases and/or diabetes mellitus, **characterized in that** in a first step, a set of proteins is detected and measured by surface-enhanced laser desorption/ionization in time-of-flight mass spectrometry (SELDI-TOF-MS) in tears of an individual, and, in a second step, the protein pattern is correlated with corresponding patterns of healthy individuals and dry-eye, related ocular surface disease or diabetes mellitus patients.

2. The method according to claim 1 for the diagnosis of dry-eye disease.

3. The method according to claim 1 for the diagnosis of ocular pathologies caused by contact lenses and/or contact lens cleaning solutions.

4. The method according to claim 1 for the diagnosis of ocular pathologies caused by smoking.

5. The method according to claim 1 for the diagnosis of diabetes mellitus.

6. The method according to claim 1 wherein the protein pattern is correlated using univariate analysis, multivariate statistical techniques and/or artificial neural networks.

7. The method according to claim 1 wherein the protein pattern consists of between 3 to 30 marker proteins.

8. The method according to claim 1 wherein the protein pattern consists of between 5 to 10 marker proteins.

9. The method according to claim 1 wherein the protein pattern comprises 7 marker proteins with an approximate molecular weights of 3700 Da, 3916 Da, 4027 Da, 4052 Da, 4136 Da, 5792 Da and 10834 Da.

10. The method according to claim 1 wherein the protein pattern comprises 4, 5 or 6 marker proteins out of the 7 marker proteins of molecular weights of 3700 Da, 3916 Da, 4027 Da, 4052 Da, 4136 Da, 5792 Da and 10834 Da.

11. The method according to claim 1 wherein the change in the protein pattern over time is used to assess the progression and/or severeness of dry-eye, related ocular surface diseases and/or diabetes mellitus.

12. The method according to claim 1 wherein the change in the protein pattern over time is used to assess the success of a therapeutic treatment.

13. A kit for the diagnosis of dry-eye, related ocular surface diseases and/or diabetes mellitus according to claim 1, comprising test strips, materials for lab-on-a-chip approaches, ELISA techniques or mass spectrometry approaches, or biochips, and, optionally, a bundled computer software including the specialized algorithm to detect said diseases in the protein pattern of patients.
